(19) Europäisches Patentamt
European Patent Office
Office européen des brevets

(11) Numéro de publication: **0 122 827**
**B1**

(12) # FASCICULE DE BREVET EUROPÉEN

(45) Date de publication du fascicule du brevet: **23.07.86**

(21) Numéro de dépôt: **84400490.3**

(22) Date de dépôt: **12.03.84**

(51) Int. Cl.⁴: **C 07 C 103/78**, C 07 C 103/30, C 07 C 149/41, A 61 K 31/00 // C07D303/22

(54) **(Carbéthoxy-2 benzalkylamido-4 phénoxy)-1 amino-3 propanols-2, leurs préparations et leurs utilisations en thérapeutique.**

(30) Priorité: **14.03.83 FR 8304150**
**21.11.83 FR 8318509**

(43) Date de publication de la demande: **24.10.84 Bulletin 84/43**

(45) Mention de la délivrance du brevet: **23.07.86 Bulletin 86/30**

(84) Etats contractants désignés: **AT BE CH DE FR GB IT LI LU NL SE**

(56) Documents cité: **US-A-3 852 468**

(73) Titulaire: **CARPIBEM (CENTRE D'ACTIVITE ET DE RECHERCHE PHARMACEUTIQUE INDUSTRIELLE BIOLOGIQUE ET MEDICALE) Société Anonyme dite:, 128, rue Danton, F-92500 Rueil- Malmaison (FR)**

(72) Inventeur: **Bouley, Etienne, 9, rue Moguez, F-92210 Saint- Cloud (FR)**
Inventeur: **Lacrampe, Jean, 14, allée de Saint Cucufa, F-92420 Vaucresson (FR)**
Inventeur: **Teulon, Jean- Marie, 12 Résidence Bel Ebat, F-78170 La Celle Saint Cloud (FR)**

(74) Mandataire: **Combe, André, CABINET BEAU DE LOMENIE 55 rue d'Amsterdam, F-75008 Paris (FR)**

EP 0 122 827 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

La présente invention concerne en tant que produits nouveaux les dérivés du benzalkylamido-4 carbalkoxy-2 phénoxy aminopropanol de formule générale (I) et leurs sels d'addition d'acides. Les dérivés en question présentent un profil pharmacologique très original et très surprenant puisqu'ils sont doués de propriétés diurétiques, associées pour certains à des propriétés $\beta_1$ bloquantes.

La présente invention concerne également le procédé de préparation desdits produits et leur application en thérapeutique. Elle concerne également les composés intermédiaires nouveaux permettant la synthèse desdits produits.

Dans l'artantérieur, sont déjà connus du brevet US—A—3852468 (Horve), des produits de structure proche, ayant des propriètés β bloquantes.

Les nouveaux composés selon l'invention appartiennent à l'ensemble constitué par les produits de formule générale (I) et leurs sels d'addition d'acides non toxiques

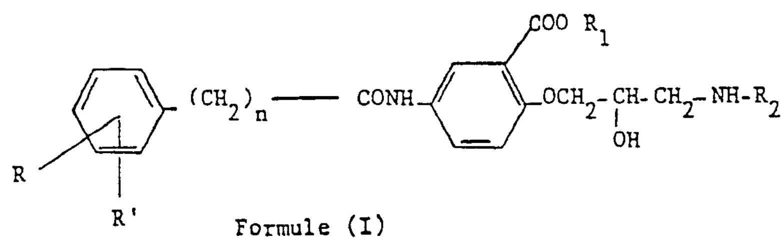

Formule (I)

Dans la formule (I):

—R et R' peuvent chacun désigner un atome d'hydrogène, un atome d'halogène par exemple le fluor, un groupe nitro ou des radicaux alkyles ou alkoxy en C1—C5 ramifiés ou non, ou un groupe $SCH_3$ ou $CF_3$.

—$R_1$ représente un radical alkyle inférieur de 1 à 5 atomes de carbone, ramifié ou non, avantageusement le radical éthyle;

—$R_2$ représente un radical alkyle inférieur de 1 à 5 atomes de carbone, linéaire ou ramifié, et de préférence le radical isopropyle ou le radical terbutyle;

—n désigne un entier inférieur à 5, et de préférence est égal à 0 ou 1.

Les composés selon l'invention sont synthétisés par action d'une base $NH_2$—$R_2$ ($R_2$ étant défini comme ci-dessus) sur un composé de formule (II) sans solvant ou dans un solvant organique usuel comme les alcools, à une température comprise entre 20°C et 150°C.

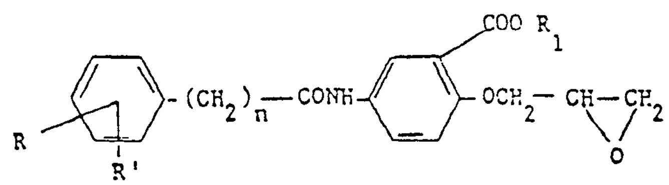

Formule (II)

Dans la formule (II), R, R' et $R_1$ et n sont définis comme ci-dessus.

D'une façon générale, les composés de formule (II) sont obtenus par réaction d'un phénol de formule générale (III) avec un excès d'épihalogénhydrine, notamment l'épichlorhydrine ou l'épibromhydrine, en présence d'un catalyseur comme le chlorure de benzyltriméthylammonium, à une température allant de 110°C à 130°C. Ce procédé fournit des composés parfaitement cristallisés avec de meilleurs rendements que les procédés évoqués ci-après. Les composés de formule (II) peuvent aussi être obtenus par réaction du phénol de formule générale (III) avec une épihalogénhydrine quelconque selon les procédés usuels. Le phénol de formule (III) sera alors métallé par des agents usuels de métallation comme la potasse, la soude, un alcoolate ou l'hydrure de sodium dans un solvant hydroalcoolique, alcoolique ou le diméthylformamide (DMF) à une température comprise entre 20°C et 150°C. Ces procédés toutefois donnent des produits moins bien définis.

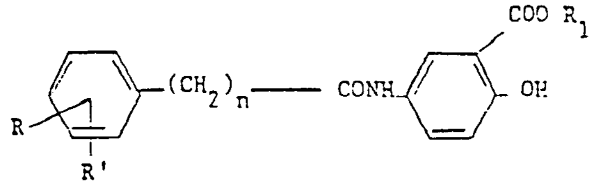

Formule (III)

Dans la formule (III) R, R' et $R_1$ et n sont définis comme ci-dessus.

Les composés de formule (III) s'obtiennent suivant des procédés connus de préparation consistant soit

à faire réagir le chlorure de l'acide arylalkanoïque correspondant avec un amino-4 carbalkoxy-2 phénol en présence d'une base comme la triéthylamine dans un solvant comme l'acétone ou le benzène, soit à estérifier l'acide (IV) correspondant en milieu acide sulfurique-alcool.

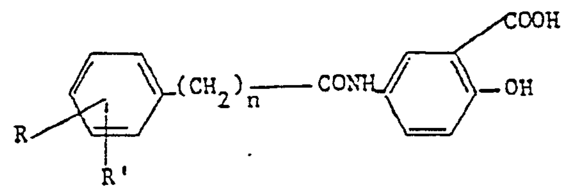

Formule (IV)

Dans la formule (IV) R, R' et n sont définis comme ci-dessus.

Les sels d'addition des composés de formule (I) peuvent s'obtenir par réaction de ces composés avec un acide minéral ou organique suivant une méthode connue en soi. Parmi les acides utilisables à cet effet on citera notamment les acides chlorhydrique, bromhydrique, acétique, p-toluène sulfonique, méthane sulfonique, oxalique, succinique, maléique, fumarique, cinnamique, malique, aspartique, glutamique, ascorbique, N-acétyl aspartique, N-acétyl glutamique.

Les nouveaux composés selon l'invention possèdent des propriétés pharmacologiques remarquables et peuvent être utilisés en thérapeutique pour le traitement de l'hypertension puisqu'ils présentent, et ceci de manière très suprenante, des propriétés diurétiques avec, pour certains des produits, des propriétés $\beta_1$ bloquantes, et une faible toxicité. Ces caractères remarquables semblent être dus à la présence dans les molécules de la fonction ester en ortho et de la fonction amide en para.

En thérapeutique humaine, les composés de formule (I) et leurs sels d'addition d'acides non toxiques peuvent être administrés notamment par voie orale. On préconise alors l'emploi de gélules ou de comprimés renfermant de 50 à 300 mg de principe actif en association avec un excipient physiologiquement acceptable. Les composés revendiqués présentent l'avantage de rendre le traitement plus aisé.

D'autre part, par rapport aux associations β-bloqueurs-diurétiques utilisées dans le traitement de l'hypertension, les composés de formule (I) ont l'avantage d'une pharmacocinétique unique. Comme autres exemples d'indications possibles, on citera l'angor et l'arythmie.

D'autres caractéristiques et avantages de l'invention apparaîtront mieux à la lecture de quelques exemples de préparation non limitatifs donnés à titre d'illustration.

Les formules correspondant aux exemples sont données dans le tableau unique en fin de texte.

## Exemple 1

[Carbéthoxy-2 (fluoro-4 benzamido)-4 phénoxy]-1 époxy-2,3 propane

Formule II R = Fluor en 4 $R_1$ = $C_2H_5$

R' = H n = O

Dans un ballon, le mélange de 34 g de carbéthoxy-2 (fluoro-4 benzamido)-4 phénol et 150 ml d'épichlorhydrine est chauffé jusqu'à 110°C. Puis 2,7 g de chlorure de benzyltriméthylammonium sont alors introduits. Le mélange réactionnel est chauffé à reflux pendant ½ h puis refroidi. Une fois la température redescendue à 50°C, 150 ml d'eau sont ajoutés et le mélange bien agité. La phase organique est décantée et la phase aqueuse extraite par 2 fois 50 ml d'éther. Les phases organiques sont séchés sur sulfate de magnésium puis concentrées sous vide. Le résidu brut obtenu cristallise dans 200 ml d'éther pour donner 22 g de [carbéthoxy-2 (fluoro-4 benzamido)-4 phénoxy]-1 époxy-2,3 propane fondant à 115°C.

## Exemple 2

[Carbéthoxy-2 (fluoro-3 benzamido)-4 phénoxy]-1 époxy-2,3 propane

Formule II R = Fluor en 3 $R_1$ = $C_2H_5$

R' = H n = O

Le mode opératoire est identique à celui décrit dans l'exemple 1. A partir de 50 g de carbéthoxy-2 (fluoro-3 benzamido)-4 phénol, 4 g chlorure de benzyltriméthylammonium et 250 ml d'épichlorhydrine, on obtient 24 g de [carbéthoxy-2 (fluoro-3 benzamido)-4 phénoxy]-1 époxy-2,3 propane fondant à 100°C.

Exemple 3

[Carbéthoxy-2 (fluoro-2 benzamido)-4 phénoxy]-1 époxy-2,3 propane

Formule II  R = Fluor en 2  $R_1 = C_2H_5$

$R' = H$  $n = O$

Le mode opératoire est le même que celui décrit à l'exemple 1. A partir de 36 g de carbéthoxy-2 (fluoro-2 benzamido)-4 phénol, 3 g de chlorure de benzyltriméthylammonium et 185 ml d'épichlorhydrine, on obtient 14 g de [carbéthoxy-2 (fluoro-2 benzamido)-4 phénoxy]-1 époxy-2,3 propane fondant à 89°C.

Exemple 4

[Carbéthoxy-2 (fluoro-4 benzamido)-4 phénoxy]-1 terbutylamino-3 propanol-2

$CH_3$

Formule I  R = Fluor en 4  $R_1 = C_2H_5$  $R_2 = —C—CH_3$

$CH_3$

$R' = H$  $n = O$

Le mélange de 11 g du produit de l'exemple 1, 20 ml de terbutylamine et 60 ml d'éthanol est chauffé à 60°C pendant 8 h, puis concentré sous vide. Le résidu est repris dans 3 ml d'acide acétique dans 200 ml d'eau et 200 ml d'acétate d'isopropyle. Le mélange est bien agité et l'acétate d'isopropyle éliminé par décantation. La phase aqueuse est alors neutralisée par l'ammoniaque puis extraite par 3 fois 50 ml de chlorure de méthylène. Aprés séchage sur sulfate de magnésium et concentration sous vide de la phase organique, on obtient un résidu qui cristallise dans l'éther. La recristallisation dans l'acétate d'isopropyle fournit 8 g de [carbéthoxy-2 (fluoro-4 benzamido)-4 phénoxy]-1 terbutylamino-3 propanol-2 sous forme de cristaux blancs fondant à 130°C.

Exemple 5

[Carbéthoxy-2 (fluoro-4 benzamido)-4 phénoxy]-1 isopropylamino-3 propanol-2

$CH_3$

Formule I  R = Fluor en 4  $R_1 = C_2H_5$  $R_2 = —CH$

$CH_3$

$R' = H$  $n = O$

Le mode opératoire est lemême que celui décrit à l'exemple 4 mais en utilisant l'isopropylamine à la place de la terbutylamine. A partir de 11 g du produit de l'exemple 1, on obtient un produit brut qui recristallisé dans l'acétate d'isopropyle fournit 6 g de [carbéthoxy-2 (fluoro-4 benzamido)-4 phénoxy]-1 isopropylamino-3 propanol-2 sous forme de cristaux blancs fondant à 117°C.

Exemple 6

[Carbéthoxy-2 (fluoro-3 benzamido)-4 phénoxy]-1 terbutylamino-3 propanol-2

$CH_3$

Formule I  R = Fluor en 3  $R_1 = C_2H_5$  $R_2 = —C—CH_3$

$CH_3$

$R' = H$  $n = O$

Le mode opératoire est le même que celui décrit à l'exemple 4. A partir de 14 g du produit de l'exemple 2 et de 80 ml de terbutylamine, on obtient après recristallisation dans l'acétate d'isopropyle 7 g de [carbéthoxy-2 (fluoro-3 benzamido)-4 phénoxy]-1 terbutylamino-3 propanol-2 sous forme de cristaux blancs fondant à 114°C.

Exemple 7

Chlorhydrate de [carbéthoxy-2 (fluoro-3 benzamido)-4 phénoxy]-1 isopropylamino-3 propanol-2

Formule I  R = Fluor en 3  $R_1 = C_2H_5$  $R_2$ = 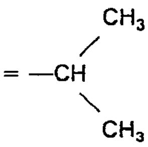

R' = H  n = O

Le mode opératoire est le même que celui décrit à l'exemple 4 mais à partir de 8 g du produit de l'exemple 2 et 30 ml d'isopropylamine, on obtient un produit blanc qui cristallise dans l'éther. En dissolvant ce produit dans l'acétone (200 ml) et en ajoutant de l'éther chlorhydrique jusqu'à pH 2, le chlorhydrate attendu précipite. On obtient ainsi après lavage à l'acétone et séchage 3 g de chlorhydrate de [carbéthoxy-2 (fluoro-3 benzamido)-4 phénoxy]-1 isopropylamino-3 propanol-2 sous forme de cristaux blancs fondant à 182°C.

Exemple 8

[Carbéthoxy-2 (fluoro-2 benzamido)-4 phénoxy]-1 terbutylamino-3 propanol-2

Formule I  R = Fluor en 2  $R_1 = C_2H_5$  $R_2$ = 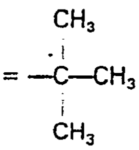

R' = H  n = O

Le mode opératoire est le même que celui décrit à l'exemple 4. En partant de 12 g du produit de l'exemple 3, on obtient 3,5 de [carbéthoxy-2 (fluoro-2 benzamido)-4 phénoxy]-1 terbutylamino-3 propanol-2 sous forme de cristaux blancs fondant à 124°C.

Exemple 9

Chlorhydrate de [carbéthoxy-2 (fluoro-2 benzamido)-4 phénoxy]-1 isopropylamino-3 propanol-2

Formule I  R = Fluor en 2  $R_1 = C_2H_5$  $R_2$ = 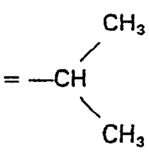

R' = H  n = O

Le mode opératoire est le même que celui décrit à l'exemple 7 mais à partir de 14 g du produit de l'exemple 3. On obtient ainsi 2 g de chlorhydrate de [carbéthoxy-2 (fluoro-2 benzamido)-4 phénoxy]-1 isopropylamino-3 propanol-2 sous forme de cristaux blancs fondant à 188°C.

Exemple 10

[Carbéthoxy-2 benzamido-4 phénoxy]-1 époxy-2,3.propane

Formule II  R = R' = H  $R_1 = C_2H_5$

n = O

Le mode opératoire est le même que celui décrit à l'exemple 1 mais à partir de 42 g de carbéthoxy-2 benzamido-4 phénol, 275 ml d'épichlorhydrine et 3,5 g de chlorure de benzyltriméthylammonium, on obtient 23 g de (carbéthoxy-2 benzamido-4 phénoxy)-1 époxy-2,3 propane sous forme de cristaux blancs fondant à 129°C.

Exemple 11

[Carbéthoxy-2 benzamido-4 phénoxy]-1 terbutylamino-3 propanol-2

Formule I $R = R' = H$ $R_1 = C_2H_5$

$n = O$

$R_2 =$ 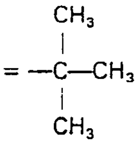

Le mode opératoire est le même que celui décrit à l'exemple 4. En partant de 10 g du produit de l'exemple 10 et de 50 ml de terbutylamine, on obtient 3,5 g de [carbéthoxy-2 benzamido-4 phenoxy]-1 terbutylamino-3 propanol-2 sous forme de cristaux blancs fondant à 120°C.

Exemple 12

[Carbéthoxy-2 (méthyl-4 benzamido)-4 phénoxy]-1 époxy-2,3 propane

Formule II $R = CH_3$ en 4 $R' = H$

$R_1 = C_2H_5$ $n = O$

Le mode opératoire est le même que celui décrit à l'exemple 1. A partir de 27 g de carbéthoxy-2 (méthyl-4 benzamido)-4 phénol, 40 ml d'épichlorhydrine et 2,3 g de chlorure de benzyltriméthylammonium, on obtient 13,1 g de [carbéthoxy-2 (méthyl-4 benzamido)-4 phénoxy]-1 époxy-2,3 propane sous forme de cristaux blancs fondant à 117°C.

Exemple 13

[Carbéthoxy-2 (méthyl-4 benzamido)-4 phénoxy]-1 terbutylamino-3 propanol-2

Formule I $R = CH_3$ en 4 $R_1 = C_2H_5$

$R' = H$ $R_2 =$ 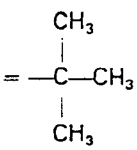

$n = O$

Le mode opératoire est le même que celui décrit à l'exemple 4, mais en partant de 11 g du produit de l'exemple 12 et de 60 ml de terbutylamine, on obtient 6,6 g de [carbéthoxy-2 (méthyl-4 benzamido)-4 phénoxy]-1 terbutylamino-3 propanol-2 sous forme de cristaux blancs fondant à 140°C,

Exemple 14

[Carbéthoxy-2 (méthyl-4 benzamido)-4 phénoxy]-1 isopropylamino-3 propanol-2

Formule I $R = CH_3$ en 4 $R_1 = C_2H_5$

$R' = H$ $R_2 =$ 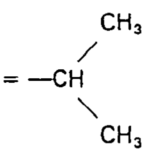

$n = O$

Le mode opératoire est le même que celui décrit à l'exemple 4, mais en remplaçant la terbutylamine par l'isopropylamine. En partant de 15 g du produit de l'exemple 12 et de 70 ml d'isopropylamine, on obtient 9 g de [carbéthoxy-2 (méthyl-4 benzamido)-4 phénoxy]-1 isopropylamino-3 propanol-2 sous forme de cristaux blancs fondant à 114°C.

Exemple 15

[Carbéthoxy-2 (méthoxy-4 benzamido)-4 phénoxy]-1 époxy-2,3 propane

Formule II  R = $OCH_3$ en 4  $R_1 = C_2H_5$

R' = H  n = O

Le mode opératoire est le même que celui décrit à l'exemple 1 mais à partir de 13,1 g de carbéthoxy-2 (méthoxy-4 benzamido)-4 phénol, de 66 ml d'épichlorhydrine et de 1 g de chlorure de benzyltriméthylammonium, on obtient 8,6 g de [carbéthoxy-2 (méthoxy-4 benzamido)-4 phénoxy]-1 époxy-2,3 propane sous forme de cristaux blancs fondant à 126°C,

Exemple 16

[Carbéthoxy-2 (méthoxy-4 benzamido)-4 phénoxy]-1 terbutylamino-3 propanol-2

Formule I  R = $OCH_3$ en 4  $R_1 = C_2H_5$

R' = H  $R_2$ = 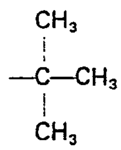

n = O

Le mode opératoire est le même que celui décrit à l'exemple 4. A partir de 8,6 g du produit de l'exemple 15 et 20 ml de terbutylamine, on obtient 5,2 g de [carbéthoxy-2 (méthoxy-4 benzamido)-4 phénoxy]-1 terbutylamino-3 propanol-2 sous forme de cristaux blancs fondant à 124°C.

Exemple 17

[Carbéthoxy-2 (difluoro-2,4 benzamido)-4 phénoxy]-1 terbutylamino-3 propanol-2

Formule I  R = Fluor en 2  $R_1 = C_2H_5$

R' = Fluor en 4  $R_2$ = 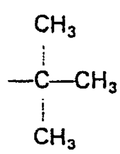

n = O

Ce produit est synthétisé de la même manière. A partir du [carbéthoxy-2 (difluoro-2,4 benzamido)-4 phénoxy]-1 époxy-2,3 propane et de la terbutylamine, on obtiendra conformément aux modes opératoires précédemment décrits le [carbéthoxy-2 difluoro-2,4 benzamido)-4 phénoxy]-1 terbutylamino-3 propanol-2. F = 138—140°C.

Exemple 18

[Carbéthoxy-2 (fluoro-4 benzacétamido)-4 phénoxy]-1 terbutyl amino-3 propanol-2

Formule I  R = Fluor en 4  $R_1 = C_2H_5$

R' = H  $R_2$ = 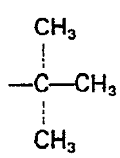

n = 1

De la même manière, le [carbéthoxy-2 (fluoro-4 benzacétamido)-4 phénoxy]-1 terbutylamino-3 propanol-2 s'obtient à partir du [carbéthoxy-2 (fluoro-4 benzacétamido)-4 phénoxy]-1 époxy-2,3 propane et de la terbutylamine suivant un mode opératoire identique à ceux décrits précédemment. F = 109°C.

Exemple 19

[carbéthoxy-2 (chloro-4 benzamido)-4 phénoxy]-1 époxy-2,3 propane

Formule II  R = Cl en 4  $R_1 = C_2H_5$

$R' = H$  $n = O$

Le mode opératoire est identique à celui décrit à l'exemple 1. A partir de 32 g de carbéthoxy-2 (chloro-4 benzamido)-4 phénol, 140 ml d'épichlorhydrine et 3 g de chlorure de benzyl triméthyl ammonium, on obtient 13,2 g de [carbéthoxy-2 (chloro-4 benzamido)-4 phénoxy]-1 époxy-2,3 propane utilisé ainsi pour la suite de la synthèse.

Exemple 20

[carbéthoxy-2 (chloro-4 benzamido)-4 phénoxy]-1 terbutylamino-3 propanol-2

$CH_3$

Formule I  R = Cl en 4  $R_1 = C_2H_5$  $R_2 = —C—CH_3$

$CH_3$

$R' = H$  $n = O$

Le mode opératoire est le même que celui décrit à l'exemple 4 mais, à partir de 13 g de [carbéthoxy-2 (chloro-4 benzamido)-4 phénoxy]-1 époxy-2,3 propane préparé à l'exemple 19, on obtient après recristallisation dans l'isopropanol 3,6 g de [carbéthoxy-2 (chloro-4 benzamido)-4 phénoxy]-1 terbutylamino-3 propanol-2 sous forme de cristaux fondant à 134°C.

Exemple 21

[carbéthoxy-2 (méthyl-3 benzamido)-4 phénoxy]-1 époxy-2,3 propane

Formule II  $R = CH_3$ en 3  $R_1 = C_2H_5$

$R' = H$  $n = O$

Selon le mode opératoire de l'exemple 1 mais en partant de 11 g de carbéthoxy-2 (méthyl-3 benzamido)-4 phénol, de 20 $cm^2$ d'épichlorhydrine et de 1 g de chlorure de benzyl triméthylammonium, on obtient 15 g de [carbéthoxy-2 (méthyl-3 benzamido)-4 phénoxy]-1 époxy-2,3 propane sous forme d'huile utilisée ainsi.

Exemple 22

[carbéthoxy-2 (méthyl-3 benzamido)-4 phénoxy]-1 terbutylamino-3 propanol-2

$CH_3$

Formule I  $R = CH_3$ en 3  $R_1 = C_2H_5$  $R_2 = —C—CH_3$

$CH_3$

$R' = H$  $n = O$

Selon le mode opératoire de l'exemple 4 mais à partir des 15 g de l'époxyde préparé à l'exemple 21, on obtient 8,6 g de [carbéthoxy-2 (méthyl-3 benzamido)-4 phénoxy]-1 terbutylamino-3 propanol-2 sous forme de cristaux blancs fondant à 109°C.

Exemple 23

[carbéthoxy-2 (méthoxy-3 benzamido)-4 phénoxy]-1 époxy-2,3 propane

Formule II  R = MeO en 3  $R_1 = C_2H_5$

$R' = H$  $n = O$

Selon le mode opératoire de l'exemple 1 mais à partir de 16,4 g de carbéthoxy-2 (méthoxy-3 benzamido)-4 phénol, de 50 ml d'épichlorhydrine et 1,5 g de chlorure de benzyl triméthylammonium, on obtient 14,5 g de [carbéthoxy-2 (méthoxy-3 benzamido)-4 phénoxy]-1 époxy-2,3 propane sous forme de cristaux fondant à 90°C.

Exemple 24

[carbéthoxy-2 (méthoxy-3 benzamido)-4 phénoxy]-1 terbutylamino-3 propanol-2

Formule I  R = MeO en 3  $R_1 = C_2H_5$  $R_2 =$ 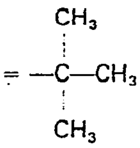

R' = H  n = O

Selon le mode opératoire de l'exemple 4 mais à partir de 14,5 g de l'époxyde préparé à l'exemple 23, on obtient 2,6 g de [carbéthoxy-2 (méthoxy-3 benzamido)-4 phénoxy]-1 terbutylamino-3 propanol-2 sous forme de cristaux blancs fondant à 94°C.

Exemple 25

[carbéthoxy-2 (méthyl-2 benzamido)-4 phénoxy]-1 époxy-2,3 propane

Formule II  R = $CH_3$ en 2  $R_1 = C_2H_5$

R' = H  n = O

Après avoir préparé le sel de sodium de 20 g de carbéthoxy-2 (méthyl-2 benzamido)-4 phénol dans l'éthanol en présence de la quantité stoéchiométrique de soude en pastille, on ajoute sous agitation 50 ml d'épichlorhydrine et on porte au reflux pendant 8 h. Le mélange réactionnel est refroidi puis concentré sous vide. Le résidu obtenu est repris dans le chlorure de méthylène et lavé à l'eau. La solution organique est séchée sur sulfate de magnésium, filtrée puis concentrée sous vide. Le résidu durcit dans l'éther isopropylique. En reprenant le solide obtenu dans un mélange de 100 ml d'éther éthylique et 10 ml d'acétate d'isopropyle, on obtient 6 g de [carbéthoxy-2 (méthyl-2 benzamido)-4 phénoxy]-1 époxy-2,3 propane sous forme de cristaux rosés fondant à 94°C.

Exemple 26

[carbéthoxy-2 (méthyl-2 benzamido)-4 phénoxy]-1 terbutylamino-3 propanol-2

Formule I  R = $CH_3$ en 2  $R_1 = C_2H_5$  $R_2 =$ 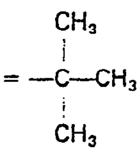

R' = H  n = O

Selon le mode opératoire de l'exemple 4 mais en partant de 13 g de l'époxyde préparé à l'exemple 25, on obtient 1,2 g de [carbéthoxy-2 (méthyl-2 benzamido)-4 phénoxy]-1 terbutylamino-3 propanol-2 sous forme de cristaux blancs fondant à 126°C.

Exemple 27

[carbéthoxy-2 (méthylthio-4 benzamido)-4 phénoxy]-1 époxy-2,3 propane

Formule II  R = $CH_3S$ en 4  $R_1 = C_2H_5$

R' = H  n = O

Selon le mode opératoire de l'exemple 25 mais à partir de 40 g de carbéthoxy-2 (méthylthio-4 benzamido)-4 phénol et 80 ml d'épichlorhydrine, on obtient 20 g de [carbéthoxy-2 (méthylthio-4 benzamido)-4 phénoxy]-1 époxy-2,3 propane sous forme de cristaux blancs fondant à 105°C.

Exemple 28

[carbéthoxy-2 (méthylthio-4 benzamido)-4 phénoxy]-1 terbutylamino-3 propanol-2

Formule I  R = $CH_3S$ en 4  $R_1 = C_2H_5$  $R_2 =$ 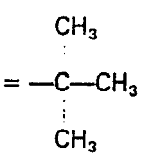

R' = H  n = O

Selon le mode opératoire de l'exemple 4 mais en partant de 12 g de l'époxyde préparé à exemple 27 et

de terbutylamine, on obtient 6 g d'un produit brut qui, recristallisé dans l'acétate d'isopropyle, fournit 3,5 g de [carbéthoxy-2 (méthylthio-4 benzamido)-4 phénoxy]-1 terbutylamino-3 propanol-2 sous forme de cristaux blancs fondant à 142°C.

Exemple 29

[carbéthoxy-2 (chlor-3 benzamido)-4 phénoxy]-1 époxy-2,3 propane

Formule II    R = Cl en 3    $R_1 = C_2H_5$

R' = H    n = O

Le mode opératoire est identique à celui de l'exemple 1. A partir de 7 g de carbéthoxy-2 (chloro-3 benzamido)-4 phénol, de 30 ml d'épichlorhydrine et de 0,7 g de chlorure de benzyl triméthylammonium, on obtient 4 g de [carbéthoxy-2 (chloro-3 benzamido)-4 phénoxy]-1 époxy-2,3 propane sous forme de cristaux blancs fondant à 95°C.

Exemple 30

[carbéthoxy-2 (chloro-3 benzamido)-4 phénoxy]-1 terbutylamino-3 propanol-2

Formule I    R = Cl en 3    $R_1 = C_2H_5$    $R_2$ = 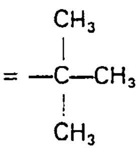

R' = H    n = O

Selon le mode opératoire décrit à l'exemple 4, 13 g de l'époxyde de l'exemple 29 fournissent 2 g de [carbéthoxy-2 (chloro-3 benzamido)-4 phénoxy]-1 terbutylamino-3 propanol-2 sous forme de cristaux blancs fondant à 107°C.

Exemple 31

Chlorhydrate de [carbéthoxy-2 (fluoro-2 benzamido)-4 phénoxy]-1 terbutylamino-3 propanol-2

Formule I    R = Fluor en 2    $R_1 = C_2H_5$

R' = H    $R_2$ = 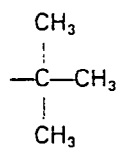

n = O
chlorhydrate

Une solution de 4,5 g de [carbéthoxy-2 (fluoro-2 benzamido)-4 phénoxy]-1 terbutylamino-3 propanol-2 préparé à l'exemple 8 dans 50 ml d'éthanol absolu, est acidifiée à pH 2 par de l'éther chlorhydrique. Le solvant est évaporé à sec sous vide, et le résidu est repris par de l'éther.

On obtient ainsi, après essorage du précipité obtenu et lavage à l'éther, 4,6 g de chlorhydrate de [carbéthoxy-2 (fluoro-2 benzamido)-4 phénoxy]-1 terbutylamino-3 propanol-2, fondant à 168°C.

Exemple 32

[carbéthoxy-2 (trifluoro méthyl-4 benzamido)-4 phénoxy]-1 époxy-2,3 propane

Formule II    R = $CF_3$ en 4    $R_1 = C_2H_5$

R' = H    n = O

Selon le mode opératoire décrit à l'exemple 25 mais à partir de 20 g de carbéthoxy-2 (trifluorométhyl-4 benzamido)-4 phénol et 50 ml d'épichlorhydrine, on obtient 7 g de [carbéthoxy-2 (trifluorométhyl-4 benzamido)-4 phénoxy]-1 époxy-2,3 propane sous forme d'une huile, que l'on utilise telle quelle pour la suite.

Exemple 33

[carbéthoxy-2 (trifluorométhyl-4 benzamido)-4 phénoxy]-1 terbutylamino-3 propanol-2

Formule I  R = $CF_3$ en 4  $R_1$ = $C_2H_5$

R' = H  $R_2$ = 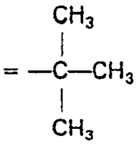

n = O

Selon le mode opératoire décrit dans l'exemple 4 mais en partant de 13 g de l'époxyde préparé à l'exemple 32 et 50 ml de terbutylamine, on obtient après cristallisation dans l'éther et lavage à l'acétate d'isopropyle 1,5 g de [carbéthoxy-2 (trifluorométhyl-4 benzamido)-4 phénoxy]-1 terbutylamino-3 propanol-2 fondant à 155°C.

Exemple 34

[carbéthoxy-2 (méthoxy-2 benzamido)-4 phénoxy]-1 époxy-2,3 propane

Formule II  R = $OCH_3$ en 2  $R_1$ = $C_2H_5$

R' = H  n = O

Selon le mode opératoire décrit à l'exemple 1 mais à partir de 8,5 g de carbéthoxy-2 (méthoxy-4 benzamido)-4 phénol, 50 ml d'épichlorhydrine et 600 mg de chlorure de benzyl triméthyl ammonium, on obtient 3,5 g de [carbéthoxy-2 (méthoxy-2 benzamido)-4 phénoxy]-1 époxy-2,3 propane cristallisés dans l'éther isopropylique et fondant à 70°C.

Exemple 35

[carbéthoxy-2 (méthoxy-2 benzamido)-4 phénoxy]-1 terbutylamino-3 propanol-2

Formule I  R = $OCH_3$ en 2  $R_1$ = $C_2H_5$

R' = H  $R_2$ = 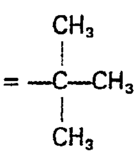

n = O

Selon le mode opératoire décrit à l'exemple 4 mais à partir de 3,5 g de l'époxyde préparé à l'exemple 34 et 50 ml de terbutylamine, on obtient 1,2 g de [carbéthoxy-2 (méthoxy-2 benzamido)-4 phénoxy]-1 terbutylamino-3 propanol-2, cristallisés dans le pentane et fondant à 107°C.

Exemple 36

[carbéthoxy-2 (chloro-2 benzamido)-4 phénoxy]-1 époxy-2,3 propane

Formule II  R = Cl en 2  $R_1$ = $C_2H_5$

R' = H  n = O

Une solution de 10 g (0,03 mole) de carbéthoxy-2 (chloro-2 benzamido)-4 phénol et 2 g de potasse à 85% (0,03 mole), dans 200 ml d'éthanol et 30 ml de DMF, est ajoutée goutte à goutte sur 4,7 $cm^3$ (0,06 mole) d'épichlorhydrine dans 50 ml d'éthanol.

Cette solution est agitée 3 h à température ambiante, puis 1 h à 50°, et laissée au repos une nuit. Puis elle est concentrée, diluée à l'eau et extraite à l'éther. Après séchage et évaporation de l'éther, on obtient 4 g d'une huile que l'on utilise telle quelle dans la suite.

Exemple 37

[carbéthoxy-2 (chloro-2 benzamido)-4 phénoxy]-1 terbutylamino-3 propanol-2 et Chlorhydrate

Formule I = R = Cl en 2 $R_1 = C_2H_5$

R' = H $R_2 =$ 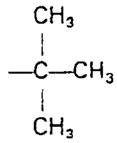

n = O

Selon le mode opératoire décrit à l'exemple 4 mais en partant de 4 g de l'époxyde préparé à l'exemple 36, on obtient 2,5 g de [carbéthoxy-2 (chloro-2 benzamido)-4 phénoxy]-1 terbutylamino-3 propanol-2 cristallisé dans un mélange acétone-éther, et fondant à 113—115°C.

*Préparation du Chlorhydrate*

6 g de [carbéthoxy-2 (chloro-2 benzamido)-4 phénoxy]-1 terbutylamino-3 propanol-2 sont dissous dans 50 ml d'acétone, et la solution est acidifiée à pH2 par l'éther chlorhydrique.

On obtient ainsi 4,5 g de chlorhydrate de [carbéthoxy-2 (chloro-2 benzamido)-4 phénoxy]-1 terbutylamino-3 propanol-2 sous forme de cristaux fondant à 158—161°.

Example 38

Chlorhydrate de [carbéthoxy-2 (difluoro-2,4 benzamido)-4 phénoxy]-1 terbutylamino-3 propanol-2

Formule I = R = Fluor en 2 $R_1 = C_2H_5$

R' = Fluor en 4 $R_2 =$ 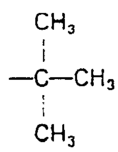

n = O

Chlorhydrate

Une solution de 25 g de [carbéthoxy-2 (difluoro-2,4 benzamido)-4 phénoxy]-1 terbutylamino-3 propanol-2 préparé à l'exemple 17 dans 100 ml d'éthanol est acidifiée à pH 2 par l'éther chlorhydrique. Le précipité formé est essoré, lavé à l'éther.

On obtient ainsi 24 g de chlorhydrate brut qui cristallisés dans l'acétonitrile, donnent 22 g de chlorhydrate de [carbéthoxy-2 (difluoro-2,4 benzamido)-4 phénoxy]-1 terbutylamino-3 propanol-2 sous forme de cristaux blancs fondant à 151—152°C.

-TABLEAU-

<u>PRODUITS EXEMPLIFIES</u>

Exemple 1 : 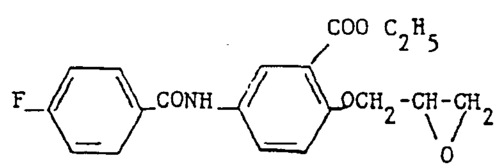

Exemple 2 : 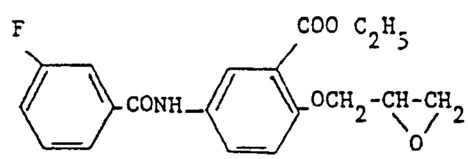

Exemple 3 : 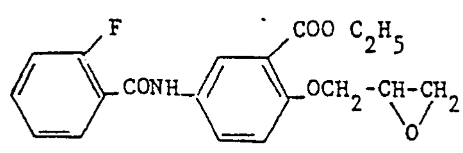

-TABLEAU-

PRODUITS EXEMPLIFIES

Exemple 4 :

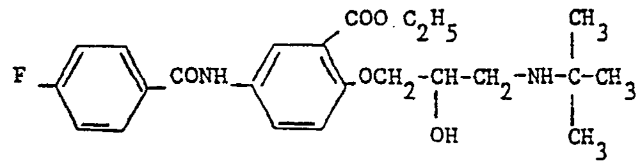

Exemple 5 :

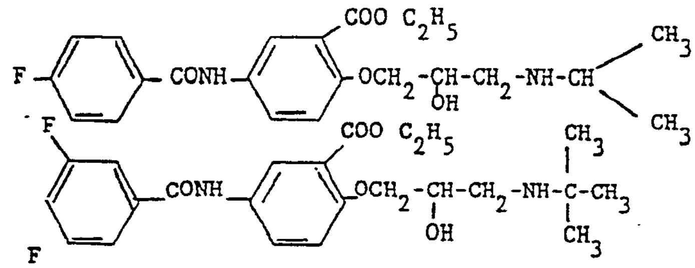

Exemple 6 :

Exemple 7 :

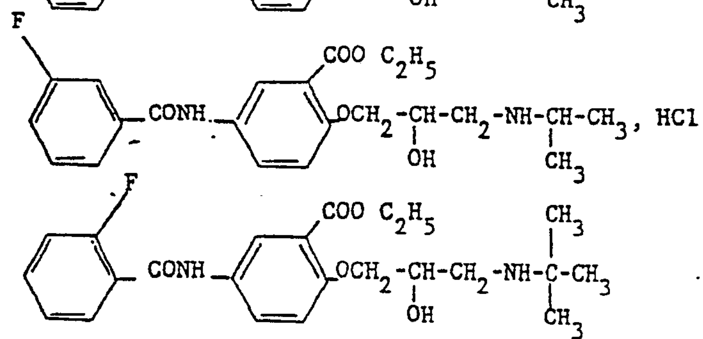

Exemple 8 :

Exemple 9 :

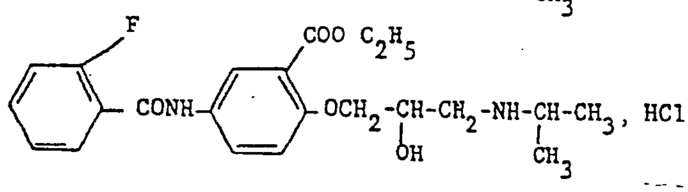

Exemple 10 :

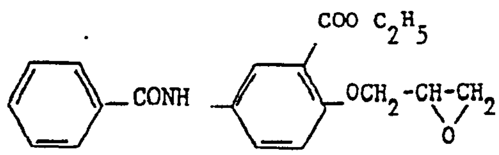

Exemple 11 :

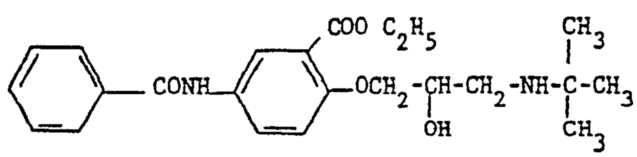

Exemple 12 :

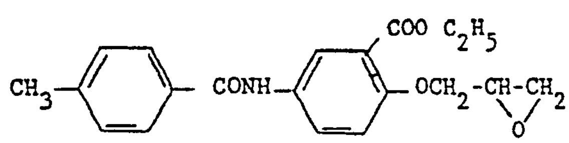

Exemple 13 :

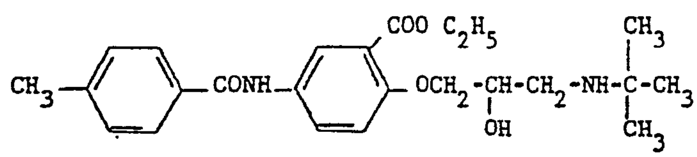

Exemple 14 :

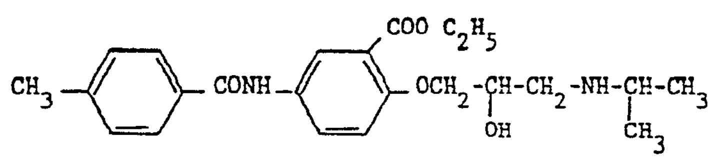

-TABLEAU-

PRODUITS EXEMPLIFIES

Exemple 15 :

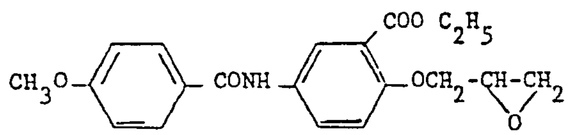

Exemple 16 :

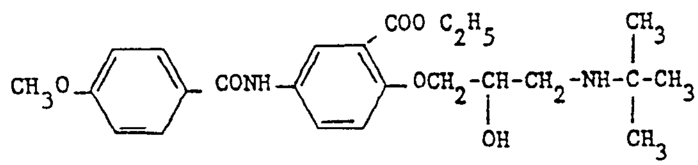

Exemple 17 :

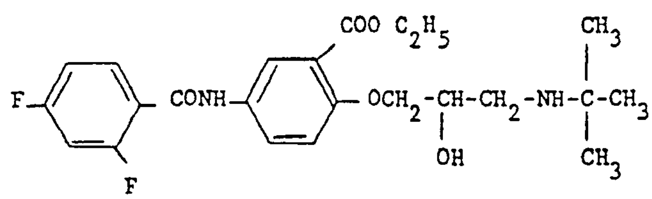

Exemple 18 :

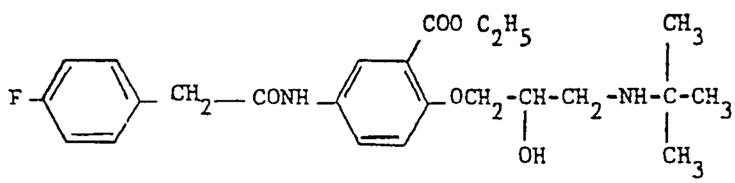

Exemple 19

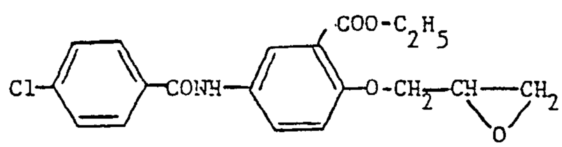

Exemple 20

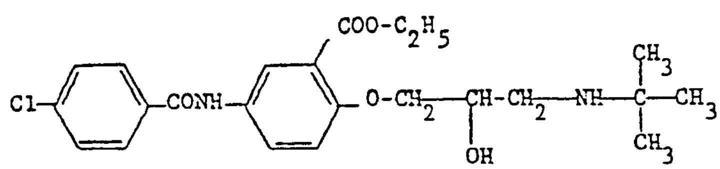

Exemple 21

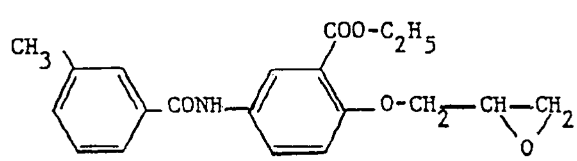

Exemple 22

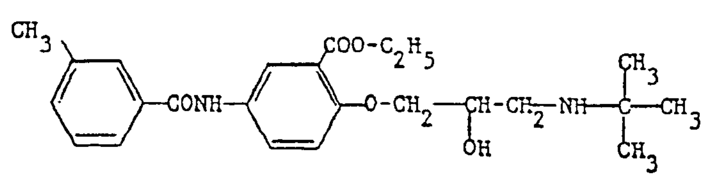

Exemple 23

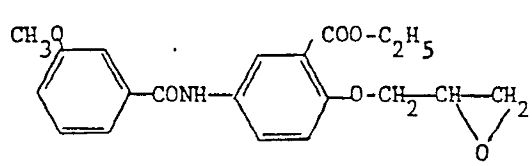

-TABLEAU-

PRODUITS EXEMPLIFIES

Exemple 24

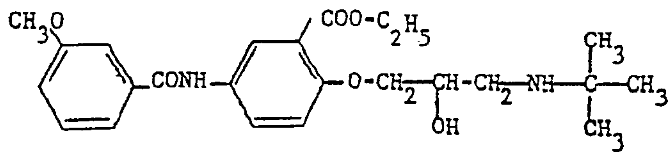

Exemple 25

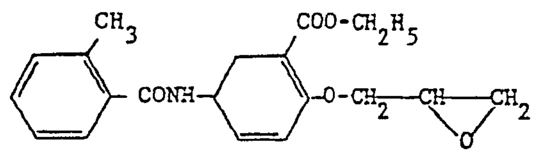

Exemple 26

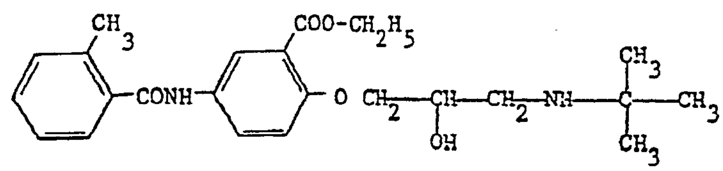

Exemple 27

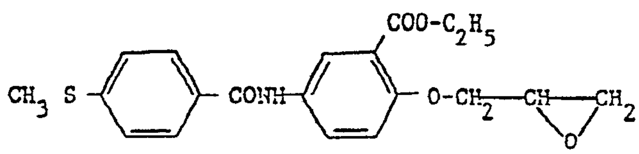

Exemple 28

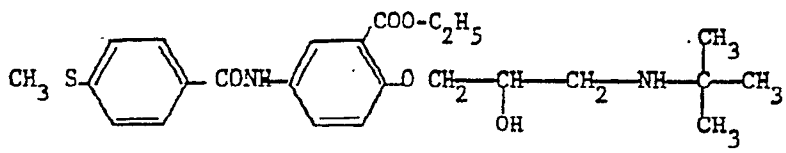

Exemple 29

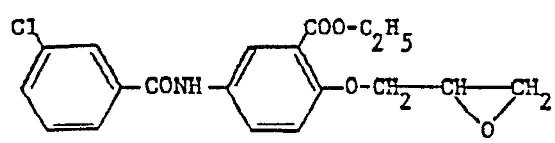

Exemple 30

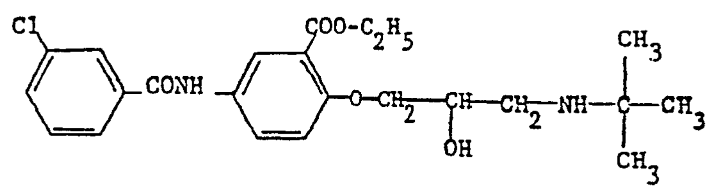

Exemple 31

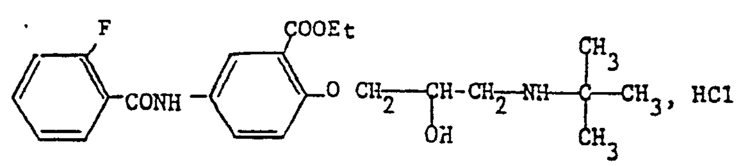

Exemple 32

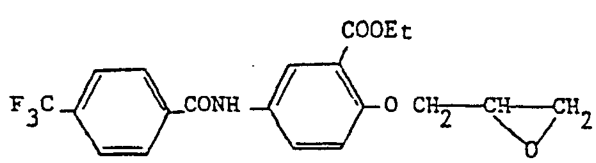

-TABLEAU-

PRODUITS EXEMPLIFIES

Exemple 33 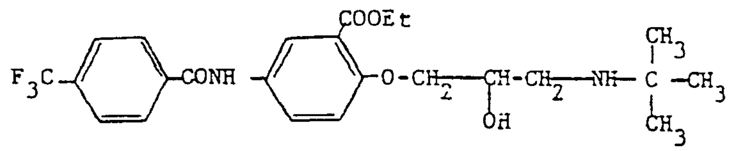

Exemple 34 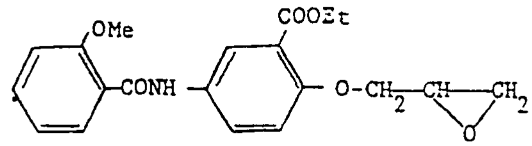

Exemple 35 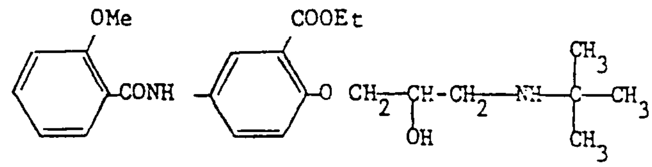

Exemple 36 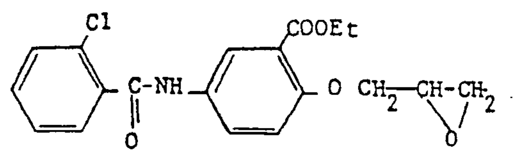

Exemple 37 : 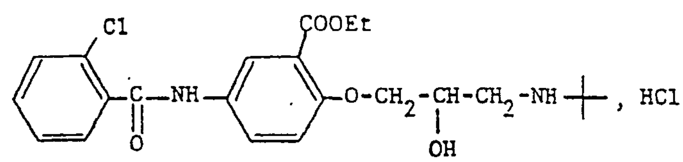

Exemple 38 : 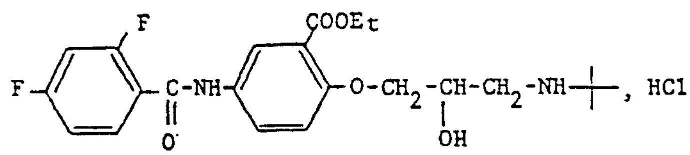

**Revendications pour les Etats contractants: BE CH DE FR GB IT LI LU NL SE**

1. Composés caractérisés en ce qu'ils répondent à la formule

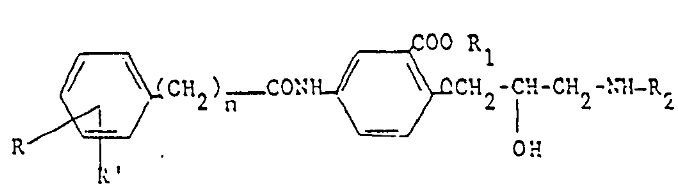

Formule (I)

dans laquelle:

— R et R' peuvent chacun désigner un atome d'hydrogène, un atome d'halogène, un groupe nitro ou des radicaux alkyles ou alkoxy en C1—C5, ramifiés ou non, ou un groupe $SCH_3$ ou $CF_3$.

— $R_1$ représente un radical alkyle inférieur en C1—C5, ramifié ou non;

— $R_2$ représente un radical alkyle inférieur en C1—C5, ramifié ou non; et

— n désigne un nombre entier inférieur à 5.

2. Composés selon la revendication 1, caractérisés en ce que $R_1$ représente le groupe éthyle.

3. Composés selon la revendication 1 ou 2, caractérisés en ce que $R_2$ représente le radical isopropyle ou terbutyle.

4. Composés selon l'une quelconque des revendications 1 à 3, caractérisés en ce que n est égal à 0 ou 1.

5. Composés selon l'une quelconque des revendications 1 à 3, caractérisés en ce que R = H et R' = F ou R = R' = F.

6. Composés selon la revendication 1, caractérisé en ce qu'il est le Chlorhydrate de [carbéthoxy-2 (difluoro-2,4 benzamido)-4 phénoxy]-1 terbutylamino-3 propanol-2.

7. Procédé de préparation des comosés selon l'une quelconque des revendications 1 à 6, caractérisé en ce que l'on fait réagir une base de formule:

$$NH_2—R$$

sur un composé de formule:

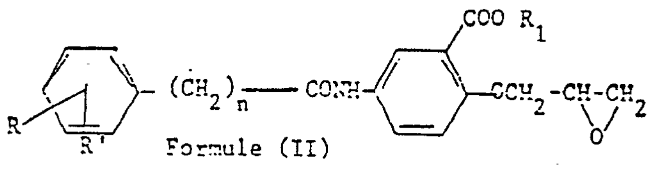

Formule (II)

R, R', $R_1$ et n étant tels que définis dans l'une quelconque des revendications 1 à 6, sans solvant ou dans un solvant organique usuel comme un alcool, à une température comprise entre 20 et 150°C.

8. Procédé selon la revendication 7, caractérisé en ce que le composé de formule (II) est préparé par réaction d'un phénol de formule:

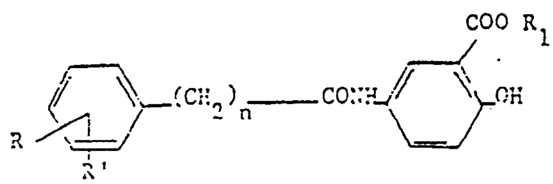

Formule (III)

sur un excès d'épihalogénhydrine comme l'épichlorhydrine ou l'épibromhydrine, en présence d'un catalyseur comme le chlorure de benzyltriméthylammonium, entre 110 et 130°C, R, R', $R_1$ et n étant tels que définis dans l'une quelconque des revendications 1 à 6.

9. Medicaments utiles pour leurs propriétés diurétiques et éventuellement $\beta_1$ bloquantes, caractérisés en ce qu'ils contiennent au moins un composé selon l'une quelconque des revendications 1 à 6.

**Revendications pour l'Etat contractant: AT**

1. Procédé de préparation de nouveaux composés répondant à la formule

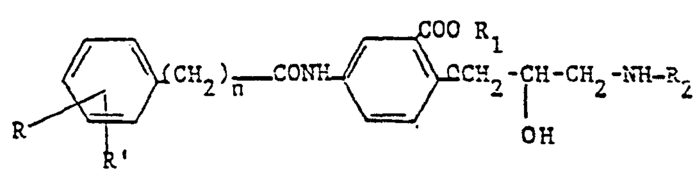

Formule (I)

dans laquelle:

— R et R' peuvent chacun désigner un atome d'hydrogène, un atome d'alogène, un groupe nitro ou des radicaux alkyles ou alkoxy en C1—C5, ramifiés ou non, ou un groupe $SCH_3$ ou $CF_3$.

— $R_1$ représente un radical alkyle inférieur en C1—C5, ramifié ou non;

— $R_2$ représente un radical alkyle inférieur en C1—C5, ramifié ou non; et

— n désigne un nombre entier inférieur à 5,

caractérisé en ce que l'on fait agir une base de formule:

$$NH_2—R_2$$

où $R_2$ est tel que défini ci-dessus sur un composé de formule (II) sans solvant ou dans un solvant organique usuel comme les alcools, à une température comprise entre 20°C et 150°C,

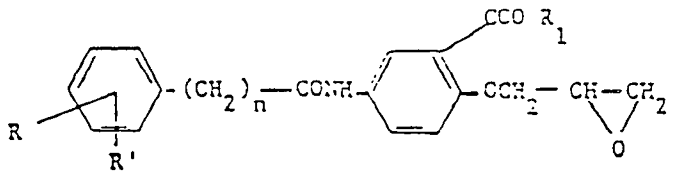

Formule (II)

R, R', $R_1$ et n étant tels que définis ci-dessus.

2. Procédé selon la revendication 1, caractérisé en ce que les composés de formule II sont obtenus par réaction d'un phénol de formule générale (III) avec un excès d'épihalogénhydrine, notamment l'épichlorhydrine ou l'épibromhydrine, en présence d'un catalyseur comme le chlorure de benzyltriméthylammonium, à une température allant de 110°C à 130°C,

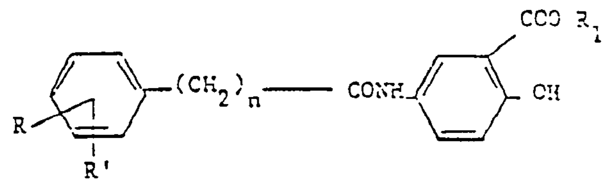

Formule (III)

R, R', n et $R_1$ étant tels que définis dans la revendication 1.

3. Procédé selon la revendication 1, caractérisé en ce que $R_1$ représente le groupe éthyle.

4. Procédé selon la revendication 1 ou 2, caractérisé en ce que $R_2$ représente le radical isopropyle ou terbutyle.

5. Procédé selon l'une quelconque des revendications 1 à 4, caractérisé en ce que n est égal à 0 ou 1.

6. Procédé selon l'une quelconque des revendications 1 à 4, caractérisé en ce que R = H et R' = F ou R = R' = F.

7. Procédé selon la revendication 1, caractérisé en ce que l'on obtient chlorhydrate de [carbéthoxy-2 (difluoro-2,4 benzamido)-4 phénoxy]-1 terbutylamino-3 propanol-2.

**Patentansprüche dür die Vertragsstaaten: BE CH DE FR GB IT LI LU NL SE**

1. Verbindungen, dadurch gekennzeichnet, daß sie der Formel

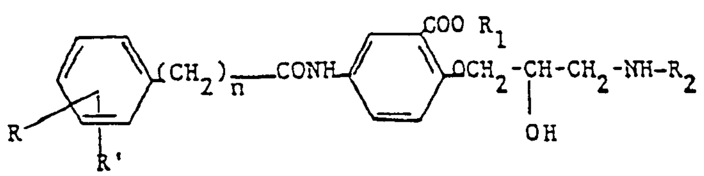

Formel (I)

entsprechen, worin:

— R und R' jeweils ein Wasserstoffatom, ein Halogenatom, eine Nitrogruppe oder eine verzweigte oder nichtverzweigte $C_1$—$C_5$-Alkyl- oder -Alkoxygruppe oder eine Gruppe $SCH_3$ oder $CF_3$ bezeichnen können;

— $R_1$ eine verzweigte oder nichtverzweigte $C_1$—$C_5$-Niedrigalkylgruppe darstellt;

— $R_2$ eine verzweigte oder nichtverzweigte $C_1$—$C_5$-Niedrigalkylgruppe darstellt; und

— n eine ganze Zahl unter 5 bedeutet.

2. Verbindungen nach Anspruch 1, dadurch gekennzeichnet, daß $R_1$ die Gruppe Äthyl darstellt.

3. Verbindungen nach Anspruch 1 oder 2, dadurch gekennzeichnet, daß $R_2$ die Gruppe Isopropyl oder tert. Butyl darstellt.

4. Verbindungen nach einem der Ansprüche 1 bis 3, dadurch gekennzeichnet daß n gleich 0 oder 1 ist.

5. Verbindungen nach einem der Ansprüche 1 bis 3, dadurch gekennzeichnet daß R = H und R' = F oder R = R' = F.

6. Verbindungen nach Anspruch 1, dadurch gekennzeichnet, daß sie 1-[2-Carbäthoxy-4-(2,4-difluorbenzamido)-phenoxy]-3-tert.butylamino-propan-2-ol hydrochlorid ist.

7. Verfahren zur Herstellung von Verbindungen nach einem der Ansprüche 1 bis 6, dadurch gekennzeichnet, daß man eine Base der Formel

$$NH_2—R$$

mit einer Verbindung der Formel

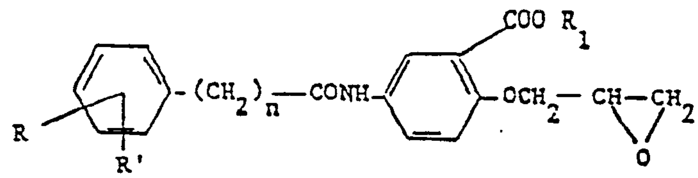

Formel (II)

worin R, R', $R_1$ und n wie in einem der Ansprüche 1 bis 6 definiert sind, ohne Lösungsmittel oder in einem üblichen organischen Lösungsmittel, wie einem Alkohol, bei einer Temperatur zwischen 20 und 150°C zur Umsetzung bringt.

8. Verfahren nach Anspruch 7, dadurch gekennzeichnet, daß die Verbindung der Formel (II) hergestellt wird durch Umsetzung eines Phenols der Formel

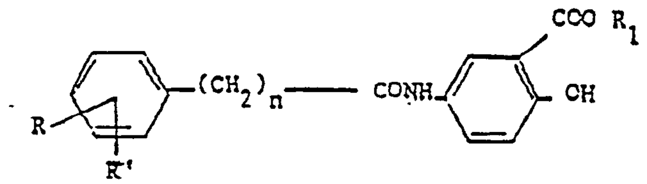

Formel (III)

mit einem überschuß an Epihalogenhydrin, wie Epichlorhydrin oder Epibromhydrin, in Gegenwart eines Katalysators, wie Benzyltrimethylammoniumchlorid, zwischen 110 und 130°C, wobei R, R', $R_1$ und n die Bedeutung wie in einem der Ansprüche 1 bis 6 haben.

9. Medikamente, die aufgrund ihrer diuretischen und gegebenenfalls $\beta_1$-blockierenden Eigenschaften nützlich sind, dadurch gekennzeichnet, daß sie mindestens eine Verbindung nach einem der Ansprüche 1 bis 6 enthalten.

**Patentansprüche für den Vertragsstaat: AT**

1. Verfahren zur Herstellung neuer Verbindungen der Formel

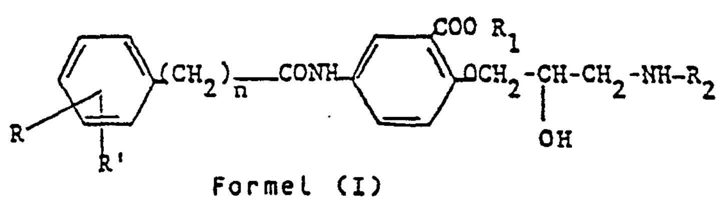

Formel (I)

worin:

— R und R' jeweils ein Wasserstoffatom, ein Halogenatom, eine Nitrogruppe oder eine verzweigte oder nichtverzweigte $C_1$—$C_5$-Alkyl- oder -Alkoxygruppe oder eine Gruppe $SCH_3$ oder $CF_3$ bezeichnen können;

— $R_1$ eine verzweigte oder nichtverzweigte $C_1$—$C_5$-Niedrigalkylgruppe darstellt;

— $R_2$ eine verzweigte oder nichtverzweigte $C_1$—$C_5$-Niedrigalkylgruppe darstellt; und

— n eine ganze Zahl unter 5 bedeutet

dadurch gekennzeichnet, daß man eine Base der Formel

$$NH_2—R_2$$

worin $R_2$ obige Bedeutung hat, mit einer Verbindung der Formel

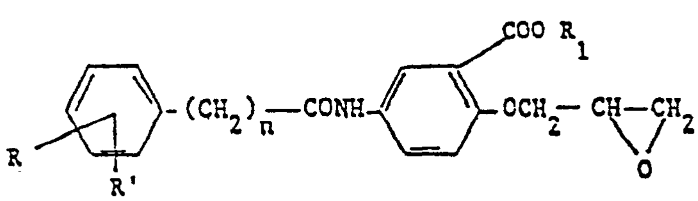

Formel (II)

worin R, R', $R_1$ und n obige Bedeutung haben, ohne Lösungsmittel oder in einem üblichen organischen Lösungsmittel, wie Alkoholen, bei einer Temperatur zwischen 20 und 150°C zur Umsetzung bringt.

2. Verfahren nach Anspruch 1, dadurch gekennzeichnet, daß die Verbindungen der Formel II erhalten werden durch Umsetzung eines Phenols der allgemeinen Formel

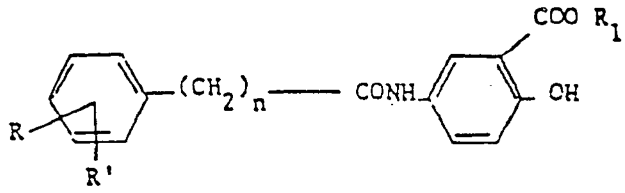

Formel (III)

worin R, R', $R_1$ und n wie in Anspruch 1 definiert sind, mit einem überschuß an Epihalogenhydrin, wie Epichlorhydrin oder Epibromhydrin, in Gegenwart eines Katalysators, wie benzyltrimethylammoniumchlorid bei einer Temperatur von 110 bis 130°C.

3. Verfahren nach Anspruch 1, dadurch gekennzeichnet, daß $R_1$ die Gruppe Äthyl darstellt.

4. Verfahren nach Anspruch 1 oder 2, dadurch gekennzeichnet, daß $R_2$ die Gruppe Isopropyl oder tert.Butyl darstellt.

5. Verfahren nach einem der Ansprüche 1 bis 4, dadurch gekennzeichnet, daß n gleich 0 oder 1 ist.

6. Verfahren nach einem der Ansprüche 1 bis 4, dadurch gekennzeichnet, daß R = H und R' = F oder R = R' = F.

7. Verfahren nach Anspruch 1, dadurch gekennzeichnet, daß man das 1-[2-Carbäthoxy-4-(2,4-difluor-benzamido)-phenoxy]-3-tert.butylamino-propan-2-ol-Hydrochlorid erhält.

**Claims for the Contracting States: BE CH DE FR GB IT LI LU NL SE**

1. Compounds characterized in that they respond to formula:

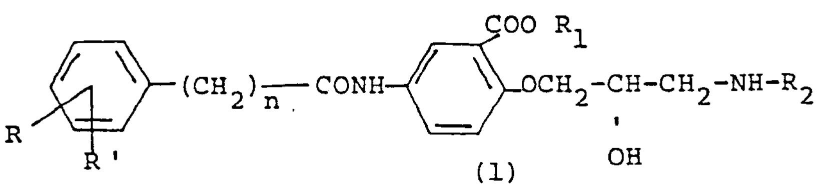

(1)

in which:

— R and R' may each designate an atom of hydrogen, an atom of halogen, a nitro group or alkyl or alkoxy radicals with C1—C5, branched or not, or a $SCH_3$ or $CF_3$ group;

— $R_1$ represents a lower alkyl radical with 1 to 5 carbon atoms, branched or not;

— $R_2$ represents a lower alkyl radical with 1 to 5 carbon atoms, branched or not; and

— n designates an integer less than 5.

2. The compounds according to claim 1, characterized in that $R_1$ represents the ethyl group.

3. The compounds according to claim 1 or 2, characterized in that $R_2$ represents the isopropyl or terbutyl radical.

4. The compounds according to any one of claims 1 to 3, characterized in that n is equal to 0 or 1.

5. The compounds according to any one of claims 1 to 3, characterized in that R = H and R' = F or R = R' = F.

6. The compound according to claim 1, characterized in that it is:
Hydrochloride of 1-[2-carbethoxy 4-(2,4-difluoro benzamido)phenoxy] 3-terbutylamino 2-propanol.

7. A process for preparing the compounds according to any one of claims 1 to 6, characterized in that it comprises the step of reacting a base of formula $NH_2$—R on a compound of formula:

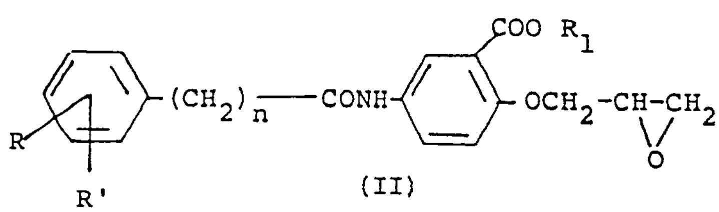

(II)

R, R', $R_1$ and n being as defined in any one of claims 1 to 6, without solvent or in a conventional organic solvent such as an alcohol, at a temperature of between 20 and 150°C.

8. The process according to claim 7, characterized in that the compound of formula (II) is prepared by reaction of a phenol of formula:

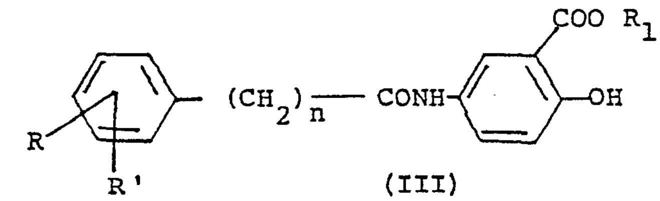

(III)

on an excess epihalohydrin such as epichlorohydrin or epibromohydrin, in the presence of a catalyst such as benzyltrimethylammonium chloride, between 110 and 130°C, R, R', $R_1$ and n being as defined in any one of claims 1 to 6.

9. Drugs useful by their diuretic and possibly $\beta_1$ blocking properties, characterized in that they contain at least one compound according to any one of claims 1 to 6.

**Claims for the Contracting State: AT**

1. A process for preparing novel compounds responding to formula

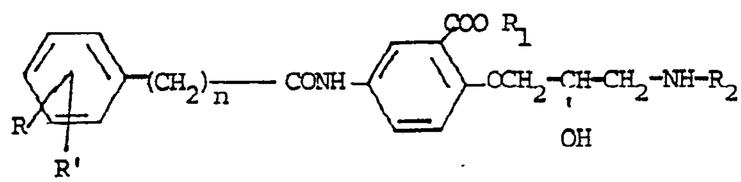

(1)

in which:

— R and R' may each designate an atom of hydrogen, an atom of halogen, a nitro group or alkyl or alkoxy radicals with C1—C5, branched or not, or a $SCH_3$ or $CF_3$ group;

— $R_1$ represents a lower alkyl radical with $C_1$—$C_5$, branched or not;

— $R_2$ represents a lower alkyl radical with $C_1$—$C_5$, branched or not; and

— n designates an integer les than 5,

characterized in that said process comprises the step of reacting a base of formula

$$NH_2—R_2$$

where $R_2$ is as defined hereinabove, on a compound of formula (II) without solvent or in a conventional organic solvent such as alcohols, at a temperature of between 20°C and 150°C,

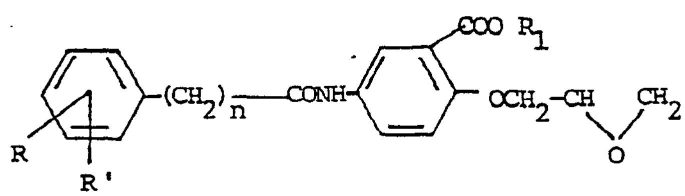

(II)

R, R', $R_1$ and n being as defined hereinabove.

2. The process according to claim 1, characterized in that the compounds of formula II are obtained by reaction of a phenol of general formula (III) with an excess of epihalohydrin, particularly epichlorohydrin or epibromohydrin, in the presence of a catalyst such as benzyltrimethylammonium chloride, at a temperature of from 110°C to 130°C

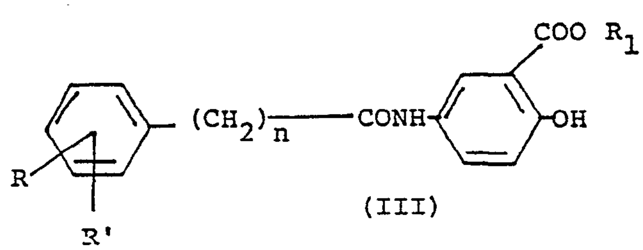

(III)

R, R' and $R_1$ being as defined in claim 1.

3. The process according to claim 1, characterized in that $R_1$ represents the ethyl group.

4. The process according to claim 1 or 2, characterized in that $R_2$ represents the isopropyl or terbutyl radical.

5. The process according to any one of claims 1 to 4, characterized in that n is equal to 0 or 1.

6. The process according to any one of claims 1 to 4, characterized in that R = H and R' = F or R = R' = F.

7. The process according to claim 1, characterized in that the Hydrochloride of 1-[2-carbethoxy 4-(2,4-difluoro benzamido) phenoxy] 3-terbutylamino 2-propanol is obtained.